# EUROPÄISCHE PATENTSCHRIFT

(11) **EP 2 915 885 B1**
(45) Veröffentlichungstag und Bekanntmachung des Hinweises auf die Patenterteilung: **11.01.2017**
(21) Anmeldenummer: 15157367.2
(22) Anmeldetag: 03.03.2015
(51) Int. Cl.: C12Q 1/68, B01D 46/00, G01N 1/22, G01N 27/447

(54) **Applikation zur PCR-Analytik von luftgetragenen Nukleinsäuren**
Application for PCR analysis of airborne nucleic acids
Application destinée à l'analyse PCR d'acides nucléiques portés par l'air

(30) Priorität: 03.03.2014 DE 102014203855
(43) Veröffentlichungstag der Anmeldung: 09.09.2015
(73) Patentinhaber: biotec Umwelt-Analytik-Beratung-Service GmbH, 33332 Gütersloh (DE)
(72) Erfinder: Bermpohl, Andreas, 33332 Gütersloh (DE)
(74) Vertreter: Grund, Martin

(56) Entgegenhaltungen:
- WO-A1-02/35209
- DE-A1- 19 814 715
- DE-C1- 19 750 215
- US-A- 5 958 111
- US-A1- 2005 260 569
- US-A1- 2010 075 313
- US-A1- 2012 199 481
- MASCLAUX F G ET AL: "Concentration of Airborne Staphylococcus aureus (MRSA and MSSA), Total Bacteria, and Endotoxins in Pig Farms", ANNALS OF OCCUPATIONAL HYGIENE, Bd. 57, Nr. 5, 5. Januar 2013 (2013-01-05) , Seiten 550-557, XP055197939, ISSN: 0003-4878, DOI: 10.1093/annhyg/mes098
- AGRANOVSKI I E ET AL: "Rapid detection of airborne viruses by personal bioaerosol sampler combined with the PCR device", ATMOSPHERIC ENVIRONMENT, PERGAMON, GB, Bd. 40, Nr. 21, 1. Juli 2006 (2006-07-01), Seiten 3924-3929, XP028076135, ISSN: 1352-2310, DOI: 10.1016/J.ATMOSENV.2006.02.023 [gefunden am 2006-07-01]
- PYANKOV O V ET AL: "Using a bioaerosol personal sampler in combination with real-time PCR analysis for rapid detection of airborne viruses", ENVIRONMENTAL MICROBIOLOGY, Bd. 9, Nr. 4, 1. April 2007 (2007-04-01), Seiten 992-1000, XP055197946, ISSN: 1462-2912, DOI: 10.1111/j.1462-2920.2006.01226.x
- JAMES J MCDEVITT ET AL: "Development of a method to detect and quantify Aspergillus fumigatus conidia by quantitative PCR for environmental air samples", MYCOPATHOLOGIA, KLUWER ACADEMIC PUBLISHERS, DO, Bd. 158, Nr. 3, 1. Oktober 2004 (2004-10-01), Seiten 325-335, XP019259800, ISSN: 1573-0832
- TOLEDANO T ET AL: "An assessment of DNA contamination risks in New York City Medical Examiner facilities.", JOURNAL OF FORENSIC SCIENCES JUL 1997, Bd. 42, Nr. 4, Juli 1997 (1997-07), Seiten 721-724, XP002741357, ISSN: 0022-1198

## Beschreibung

### Gebiet der Technik

Die vorliegende Erfindung liegt auf dem Gebiet der Bioanalytik. Genauer betrifft die Erfindung den Nachweis von luftgetragenen Nukleinsäuremolekülen.

### Hintergrund

Die Analytik von luftgetragenen Nukleinsäuremolekülen mittels klassischer oder Real-Time-PCR wird derzeit, auf Grund fehlender Probenahmetechniken und Aufbereitungsmethoden, weltweit nicht angewendet. Hingegen gäbe es verschiedene Anwendungen, die eine derartige Analytik sinnvoll erscheinen lassen.

In allen Laboren, die die PCR-Technologie routinemäßig einsetzten, taucht bei Amplifikation der gleichen Template-DNS nach einiger Zeit ein Kontaminationsproblem auf. Die regelmäßige Vervielfachung gleicher Nukleinsäurebereiche führt durch Aerosolgeneration (Pipettieren) dazu, dass die Amplifikate die Raumluft und die Wandoberflächen kontaminieren. Als Folge davon treten ab einer bestimmten Anreicherungskonzentration falsch-positive Ergebnisse auf, da die luftgetragenen Nukleinsäuren in die Ansätze neuer Analysegefäße gelangen können. Dies hat dazu geführt, dass in Laboren das Ansetzen der Mastermixe und die eigentliche PCR räumlich getrennt werden, oder aber technische Maßnahmen zum Kontaminationsschutz der Ansätze (PCR-Kabinen) zur Anwendung kommen. Derartige Nukleinsäureanreicherungen sind in Laboren der Forensik, aber auch in Laboren der Überprüfung gentechnisch veränderter Lebensmittel oder Futtermittel, zu erwarten. Im Rahmen einer vorsorgenden quantitativen Analytik scheint es also sinnvoll, routinemäßige Analysen der Raumluft auf kontaminierende Nukleinsäurefragmente durchzuführen. Neben dieser qualitätssichernden Analytik ergibt sich ein weiteres hoch interessantes Analysefeld im Rahmen der Aufnahme von forensischen Analysen an Tatorten. Jeder Mensch setzt bei Anwesenheit in einem Raum Zellmaterial und damit eindeutig identifizierbares genetisches Material in die Raumluft frei. Je nach Aktivitätsmodus wird dieses in geringerer oder höherer Konzentration freigesetzt. Abschilferungen des verhornenden mehrschichtigen Plattenepithels der Haut bei bloßer Anwesenheit, aber auch Zellmaterial, das durch Husten- bzw. Niesreflexe über die Schleimhäute in die Raumluft freigesetzt wird, enthält personenbezogene Nukleinsäureanteile. Wäre es denkbar, durch Luftfiltration eine Anreicherung dieser Materialen zu erreichen, gäbe es neben der Suche nach Zell- bzw. Blutspuren einen weiteren forensischen Ansatz, um die Anwesenheit von Menschen an einem Tatort zu beweisen. Notwendig wäre es hierzu, direkt am Tatort große Luftvolumina zu filtrieren, um möglichst viel biogenes Material der zuletzt anwesenden Personen auf einem Filter niederzuschlagen. Beispielsweise bieten die Luftsammelgeräte der Firma Sartorius die Möglichkeit, derartige Konzentrierungen von Biomolekülen auf Filteroberflächen vorzunehmen.

Aus dem Stand der Technik sind verschiedene Luftsammelvorrichtungen bekannt, z.B. in Masclaux et al. (2013): "Concentration of Airborne Staphylococcus aureus (MRSA and MSSA), Total Bacteria, and Endotoxins in Pig Farms", Annals of Occupational Hygiene, 57(5):550-557; in Agranovski et al. (2006): "Rapid detection of airborne viruses by personal bioaerosol sampler combined with the PCR device", Atmospheric Environment 40(21):3924-3929; in Pyankov et al. (2007): "Using a bioaerosol personal sampler in combination with real-time PCR analysis for rapid detection of airborne viruses", Environmental Microbiology 9(4):992-1000; und darüber hinaus in US2005/260569A1, US2005260569A1, oder US2012/199481A1.

### Kurze Beschreibung der Erfindung

Die Erfindung richtet sich auf ein Verfahren zur Analyse luftgetragener Nukleinsäuremoleküle, das Verfahren umfassend: Filtrieren und/oder Sammeln von Luft mittels eines Luftprobennahmesystems, das einen Luftfilter und/oder ein Sammelmedium umfasst, so dass die Nukleinsäuremoleküle auf dem Luftfilter und/oder im Sammelinedium zurückbleiben; Isolierung der Nukleinsäuremoleküle vom Luftfilter und/oder aus dem Sammelmedium; und Analyse der Nukleinsäuremoleküle, vorzugsweise mittels PCR und anschließender Gelelektrophorese, wobei der Filter / das Sammelmedium kontaktfrei mittels Laser oder einer mechanischen Vorrichtung in einer steril eingehausten Einheit in Einzelfragmente zerlegt wird und die Einzelfragmente manuell oder automatisch der Aufarbeitung zugeführt werden.

Die Analyse kann mittels PCR und anschließender Gelelektrophorese, mittels PCR und anschließender MALDI-TOF-Massenspektrometrie und/oder mittels *in-situ-*PCR erfolgen.

In einer Ausführungsform ist der Luftfilter ein Membranfilter aus Nitrocellulose, wobei die Porenweite vorzugsweise 5 µm bis 8 µm beträgt. In einer anderen Ausführungsform ist der Luftfilter ein Gelatinefilter, wobei die Porenweite vorzugsweise 2 µm bis 5 µm beträgt. In einer anderen Ausführungsform ist das Sammelmedium ein geeigneter Puffer, in dem die Nukleinsäuren/Zellen mittels Impaktion gesammelt werden. In einer anderen Ausführungsform stellt das Sammelmedium eine Oberfläche dar, auf der die Nukleinsäuren/Zellen elektrostatisch oder andersartig abgeschieden werden.

Das Luftprobennahmesystem ein Sartorius MD 8 System, ein Impaktionssystem oder ein elektrostatisches System sein.

In einer Ausführungsform dient das erfindungsgemäße Verfahren zum Nachweis kontaminierender Nukleinsäuremoleküle in einem Laborraum, Produktionsraum, Lagerraum, Aufenthaltsraum oder Versammlungsraum. Das Verfahren kann zur Gewinnung eines menschlichen DNS-Profils aus luftgetragenem menschlichem Zellmaterial dienen, oder auch zur forensischen Analyse von Tatorten.

In einer Ausführungsform ist das Zellmaterial Hautschuppen oder Schleimhautzellen.

Ferner umfasst die Erfindung auch die Verwendung eines Luftprobennahmesystems, das einen Luftfilter und/oder ein Sammelmedium umfasst, zur Analyse von luftgetragenen Nukleinsäuremolekülen. In einer Ausführungsform umfasst die Erfindung die Verwendung eines Luftprobennahmesystems, das einen Luftfilter und/oder ein Sammelmedium umfasst, zur Gewinnung eines menschlichen DNS-Profils aus luftgetragenem menschlichem Zellmaterial. Hierbei wird der Filter / das Sammelmedium kontaktfrei mittels Laser oder einer mechanischen Vorrichtung in einer steril eingehausten Einheit in Einzelfragmente zerlegt und die Einzelfragmente werden manuell oder automatisch der Aufarbeitung zugeführt.

### Beschreibung der Abbildungen (nachfolgend im Bildverzeichnis)

- FIG. 1: zeigt Aufbereitungsschemata für vier verschiedene Verfahren.
- FIG. 2: zeigt ein Luftprobenahmesystem MD8 des Herstellers Sartorius zur isokinetischen Probeaufnahme auf Filtrationsbasis.

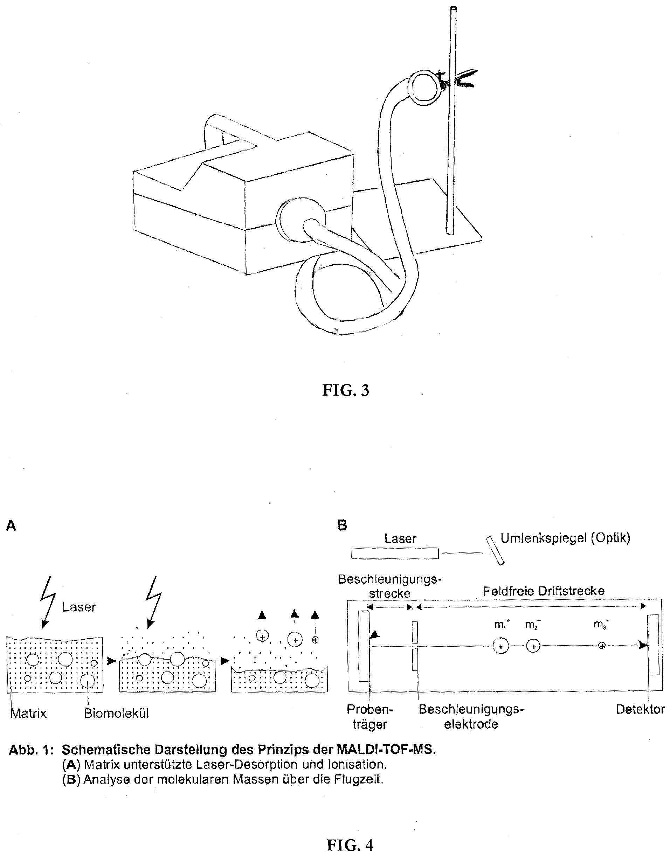

- FIG. 3: zeigt Luftfilter für das Luftprobenahmesystem MD8 des Herstellers Sartorius mit Halterungen.
- FIG. 4: zeigt schematisch das Prinzip der Analysemethode mittels MALDI-TOF MS

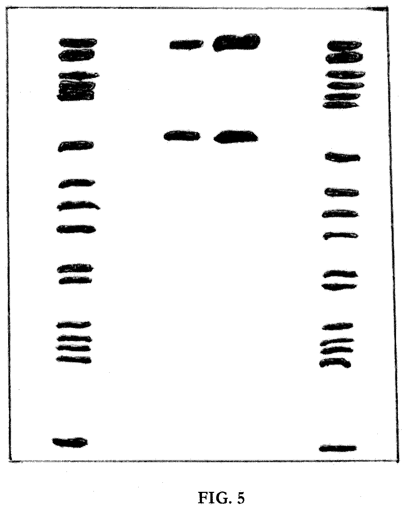

- FIG. 5: zeig das Ergebnis dieser Agarosegelelektrophorese: (v.l.n.r) Marker, Extraktion von Nitrozellulose, Extraktion von Gelatine, Marker

### Ausführliche Beschreibung

### DEFINITIONEN

Ein "Nukleinsäuremolekül", im Sinne der Erfindung, kann jedes Nukleinsäuremolekül beliebiger Länge sein. Beispiele für Nukleinsäuren sind Desoxyribonukleinsäuren (DNS), Ribonukleinsäuren (RNS) oder Peptidnukleinsäuren (PNS). Nukleinsäuren können einzelsträngig, doppelsträngig, oder teilweise einzelsträngig und teilweise doppelsträngig sein.

Ein "Luftprobenahmesystem", im Sinne der Erfindung, ist jede technische Vorrichtung, mit der nichtgasförmige Bestandteile von Luft isoliert werden können. Insbesondere werden darunter Sammel/Filtersysteme für Luft verstanden, die Partikel aus der Luft sammeln und abscheiden/auffangen können.

Ein "Tatort", im Sinne der Erfindung, ist jeglicher Ort, in geschlossenen Räumen oder außerhalb, der Schauplatz einer Tat war oder vermutlich war, dessen Hergang aufgeklärt werden soll.

Unter "forensischer Analyse" wird jede Untersuchung eines Tatortes verstanden, bei der die Feststellung der Anwesenheit eines Menschen oder anderen Lebewesens zu einem bestimmten vergangenen Zeitpunkt eine Rolle spielt.

### VERFAHREN

Die vorliegende Erfindung beruht auf der Überprüfung verschiedener Filter/Sammel - Medien/Materialien zur Luftprobenahme und der anschließenden Extraktion/Aufarbeitung der Filter-/Sammelmedien für eine nachfolgende PCR.

Ein erfindungsgemäßes Verfahren umfasst das Filtrieren/Sammeln von Luft, bei der vermutet wird, dass sie luftgetragene Nukleinsäuremoleküle enthält, mittels eines Luftprobenahmesystems, das einen Luftfilter/ein Sammelmedium umfasst, sodass Nukleinsäuremoleküle auf dem Luftfilter/Sammelmedium zurückbleiben bzw. in dem Sammelmedium verbleiben; Isolierung der Nukleinsäuremoleküle vom Luftfilter/aus dem Sammelmedium beziehungsweise Separierung der auf dem Filter/Sammelmedium niedergeschlagenen Nukleinsäuremoleküle/Zellen und Analyse der Nukleinsäuremoleküle mittels einer PCR-Reaktion. Hierbei wird der Filter / das Sammelmedium kontaktfrei mittels Laser oder einer mechanischen Vorrichtung in einer steril eingehausten Einheit in Einzelfragmente zerlegt und die Einzelfragmente werden manuell oder automatisch der Aufarbeitung zugeführt.

### Luftprobenahme

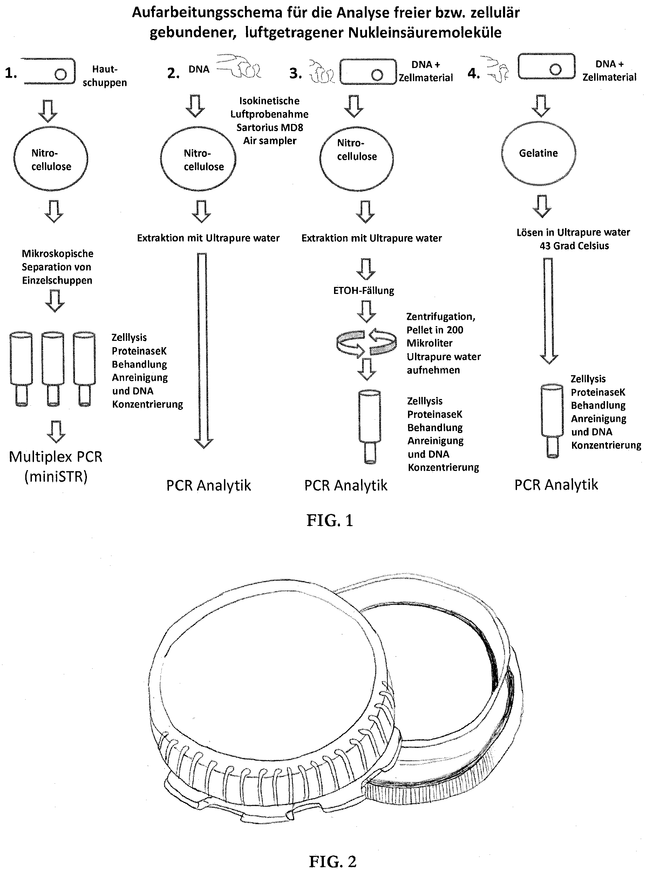

Die Luftprobenahme dient dazu, möglichst viel luftgetragene Nukleinsäure, beispielsweise als Bestandteil von biogenem Material einer Person, auf eine Filteroberfläche oder in/auf einer Matrix niederzuschlagen. Gegebenenfalls kann das Luftvolumen auch von deutlich höherem Volumen sein. In Frage kommen hierzu verschiedene Luftprobenahme-Systeme. Insbesondere bietet sich die Membranfiltration an. Bevorzugt wird ein Luftprobennahmesystem mit einer Saugleistung von mindestens 1 m³/h, optimal mindestens 3 m³/h. Eingesetzt werden kann zum Beispiel das isokinetische Probenahmesystem MD 8 der Firma Sartorius (siehe FIG. 2), die Luft wird mit einer Saugleistung von 6 m³/h durch verschiedene Filtermatrices abfiltrieren kann.

Die Zeitdauer der Luftprobenahme ist abhängig von der Konzentration der luftgetragenen Nukleinsäuren oder biogenen Materialien, die sie enthalten. Bevorzugt werden mindestens 5 m³, mindestens 10 m³, mindestens 15 m³, mindestens 20 m³, oder mindestens 25 m³ Luft durch das Luftprobennahmesystem filtriert.

Die Probenahme kann direkt mittels Stativ in, beispielsweise in 1 m Höhe, zentral im Raum erfolgen. Um bereits sedimentiertes biogenes Material zu erfassen, besteht auch die Möglichkeit, durch Ventilationstechniken Bodensediment aufzuwirbeln und der Filtration zuzuführen. Alternativ sind alle weiteren bekannten Luftprobennahmesysteme für die Probenahme denkbar: Impaktion (Austausch von Medien gegen Filter in leeren Petrischalen), Impaktion auf sonstige adsorptive Oberflächen, Impinger-Methode, elektrostatische Luftsammler, Luftwäscher, mobile RLT-Anlagen mit Sammelmedien wie Luftfilter, usw.

### Luftfilter/Sammelmedien

Geeignet als Sammelmedium/Luftfilter erwiesen sich sowohl kommerziell erhältliche Gelatinefilter als auch Nitrocellulosematrices. Bei Gelatinefiltern wird eine Porenweite von 2 µm bis 5 µm, insbesondere ca. 3 µm, bevorzugt. Bei Nitrocellulosefiltern wird eine Porenweite von 5 µm bis 8 µm bevorzugt. Alle weiteren Matrices sind jedoch ebenfalls denkbar, sofern sie an das jeweilige Probennahmesystem angepasst werden können.

### Isolierung der luftgetragenen Nukleinsäuremoleküle

Die Isolierung der Nukleinsäuremoleküle aus den Luftfiltern zur weiteren Analyse kann mit allen im Stand der Technik bekannten Mitteln vorgenommen werden.

Im Rahmen der bereits erwähnten Ausführungsform zur forensischen Analyse werden Hautschuppen z.B. vom Tatort über die Filtration auf einem Nitrocellulosefilter niedergeschlagen (siehe FIG. 1, Spalte 1).

Die Separierung besteht darin, den Filter / das Sammelmedium zu fragmentieren. Hierzu kann eine Einrichtung verwendet werden, mit der der Filter / das Sammelmedium kontaktfrei über einen Laser oder eine mechanische Vorrichtung in beliebig kleine Fragmente zerlegt werden kann. Der Laser / die mechanische Vorrichtung kann z.B. über dem Filter/Sammelmedium fixiert werden, während der Filter / das Sammelmedium z.B. auf einem xy-Kreuztisch bewegt wird. Die Bewegung des Tisches erfolgt über zwei Motorstellglieder, die im Rahmen einer Software über einen Computer angesteuert werden können. Die Gesamteinheit ist steril eingehaust (z.B. Plexiglaskubus mit steriler Luftversorgung). Über das oben beschriebene System werden Fragmente des Filters/Sammelmediums mit Einzelschuppen erhalten, die einer weiteren Analyse zugänglich sind.

Nach Separierung und Aufarbeitung dieser Hautschuppen besteht die Möglichkeit, mittels Multiplex-PCR auf STR-Profil-Basis Vollprofile zu erhalten. Hierzu muss zunächst die Zellmembran mittels Zelllyse solubilisiert werden. Dies kann in 800 µl einer frisch zubereiteten Lösung aus 0,5 mg/ml Lysozym in TE-Puffer, pH 8,0, geschehen. Nach Zugabe von 80 µl 10% SDS wird das Lysat gut gemischt und für 1-2 min im Wasserbad bei 65°C inkubiert. Dabei sollte eine klare, zähe Lösung entstehen. Anschließend werden 88 µl 1 M Natriumacetat, pH 5,2 zugegeben und gemischt. Das so entstandene Zelllysat wird daraufhin mittels Proteinase K von Proteinen befreit und darauf folgend aufgereinigt. Im letzten Schritt ist nun die DNS aufzukonzentrieren. Dies kann durch eine Ethanol/Isopropanol-Fällung geschehen. Dazu wird die vorliegende Lösung mit 0,1 Volumen 3 M Natriumacetat-Lösung (Endkonzentration 0,3 M) und 2 Volumina kaltem Isopropanol versetzt. Die ausgefällte DNS wird in der Tischzentrifuge sedimentiert und der Überstand verworfen. Das DNS-Pellet wird 1-2 mal mit 75 %-igem Ethanol gewaschen und getrocknet. Anschließend kann die DNS in destilliertem Wasser oder sterilem Puffer aufgenommen werden. Außerdem sind alle DNA Anreicherungs-/Aufreinigungsmethoden denkbar, wie Salzfällung, Phenol/Chloroform-Extraktion, Säulen und Batchverfahren.

Offenbart ist auch die Verwendung von Nitrocellulosefiltern zur direkten Detektion freier luftgetragener Nukleinsäuremoleküle. Die Filter sind einfach mit sterilem hochreinem Wasser extrahierbar und bieten damit die Möglichkeit zur direkten PCR-Analytik der luftgetragenen Nukleinsäuren (siehe FIG. 1, Spalte 2).

Offenbart ist ferner die Integrierung von zellulär gebundener DNS in die Analyse. Hierbei wird der gesamte Filter mit geringem Wasservolumen extrahiert. Anschließend ist zunächst mittels Ethanol/Isopropanol zu fällen, worauf alle freien Nukleinsäuren und Zellen mittels hochtouriger Zentrifugation pelletiert werden. Das Pellet wird dann in 200 µl hochreinem Wasser gelöst, bzw. die Zellen werden resuspendiert, und anschließend das Komplettvolumen in den weiteren Aufreinigungsschritten eingesetzt. Wie bereits für FIG. 1, Spalte 1 beschrieben, wird eine Zelllyse, Hydrolyse der Proteine und eine Aufkonzentrierung/Fällung durchgeführt (siehe FIG. 1, Spalte 3).

Offenbart ist schließlich die Verwendung von Gelatinefiltern mit Porenweiten von 3 µm als Luftfilter. In dieser Ausführungsform wird nach dem Filtrieren die komplette Matrix in hochreinem Wasser von 43 °C gelöst und anschließend die Gelatine für die PCR aus dem Ansatz entfernt, beispielsweise mittels eines NukleoSpin Food Extraktionskits (Macherey-Nagel). Kits anderer Hersteller sind hier auch einsetzbar. Das in dieser Ausführungsform eingesetzte Aufarbeitungssystem bietet aber neben der Entfernung aller proteinogenen Kontaminationen den Vorteil, dass neben der Anreicherung reiner Nukleinsäuremoleküle auch die Freisetzung und Reinigung von zellulärer Nukleinsäure möglich ist (siehe FIG. 1, Spalte 4).

### Erlangung eines STR- Vollprofils aus der isolierten Nukleinsäure

Aus der so isolierten Nukleinsäure kann anschließend ein STR-("short tandem repeat")-Profil erstellt werden. Hierzu wird in einer Ausführungsform eine STR-Multiplex-PCR durchgeführt und das entstandene Amplifikat mittels Gelelektrophorese und EDV-Auswertung analysiert (s. Beispiel 2).

### Alternativ: Aufarbeitung des Analyten mittels MALDI-TOF-MS

Die Analyse des STR-Amplifikates kann auch per MALDI-TOF-Massenspektrometrie ("matrix assisted laseer desorption/ionization - time off light) erfolgen.

Hierzu wird das, wie bereits beschrieben, mittels PCR gewonnene Amplifikat (STR-Regionen) mit einer konzentrierten Lösung der Matrix gemischt. Hierbei sollte das Matrixmolekül ein Absorptionsmaximum bei der Wellenlänge des eingestrahlten Lichts aufweisen. Für die vorherrschenden UV-Laser eignet sich in erster Linie 3-Hydroxypicolinsäure, im Besonderen für die DNS-Analyse. Nach Verdunstung des Lösemittels entsteht eine teilkristalline Schicht, in der im Idealfall die Amplifikatmoleküle durch Matrixmoleküle vollständig voneinander separiert sind. Im Vakuum des Massenspektrometers wird nun das Amplifikat auf 10 bis 20 kV aufgeladen und die Matrix mittels Laserpulsen verdampft. Durch die genaue Messung der Zeitspanne, die ein Ion (Amplifikat) benötigt, um die feldfreie, definierte Driftstrecke zwischen der Beschleunigungselektrode und dem Detektor zu durchfliegen, ist eine exakte Bestimmung der molekularen Masse des Analytmoleküls möglich.

Wie nachfolgend bei der Erlangung eines STR-Profiles beschrieben, erfolgt am Ende die Profil-zu-Person-Zuordnung anhand der Längenpolymorphismen verschiedener Genloci (STR-Regionen) unter Abgleich einer Datenbank und/oder einer Vergleichsprobe.

### Alternativ: Aufarbeitung des Analyten mittels in-situ-PCR

Ebenfalls ist eine Aufarbeitung biogenen Materials auf dem Filter selber möglich. Hierbei wird das nachfolgend in Beispiel 2 beschriebene Verfahren der STR-Profil-Erstellung aus Hautschuppen direkt auf dem Filter angewandt. Das auf dem Filter befindliche genetische Material ist hierzu mittels Paraffin zu fixieren. Bei den weiteren Verfahrensschritten, insbesondere der PCR, ist folgend darauf zu achten, dass die eingesetzten Substanzen vor Verdampfung geschützt werden.

### BEISPIELE

Beispiele, die nicht unter die Patentansprüche fallen, dienen nur der Veranschaulichung.

### Beispiel 1

### Nachweis von luftgetragenen Nukleinsäuremolekülen mittels eines Luftprobennahmesystems

Luftprobenahmesysteme mit Luftfiltern wurden darauf getestet, ob luftgetragene Nukleinsäuremoleküle mit ihnen nachgewiesen werden können. Sinnvoll ist es hierbei, beide Filtermatrices (Nitrocellulose und Gelatine) alternativ zu betrachten, da die Gelatinefilter temperatur- und feuchtigkeitsbedingte Einsatzgrenzen haben.

Die Extraktionsfähigkeit der Filtermatrices wurde durch "Spotting-Experimente" ermittelt, bei denen beide Filtermatrices mit Template-DNS bekannter Konzentration beaufschlagt wurden und anschließend eine Extraktion der Filter mit nachfolgender PCR erfolgte. Die Sensitivitätskriterien lassen sich durch diese Experimente ebenfalls bestimmen.

Um die Eignung der Filtermatrices im Rahmen der Analyse luftgetragener Nukleinsäuremoleküle zu überprüfen, wurden DNS-Aerosole durch einen Pary-Vernebler erzeugt, deren Partikelgrößen zuvor mittels Partikelmonitoring definiert wurden. Die Aerosole wurden durch ein Ventilationssystem (Luftvolumenstrom 300 m³/h) der isokinetischen Luftprobenahme mittels Membranfiltration mit einem Sartorius MD8 System zugeführt. Die Filter wurden anschließend gemäß dem folgenden Aufarbeitungsschema behandelt und einer PCR unterzogen.

Die PCR Proben wurden anschließend zur Auswertung einer Agarosegelelektrophorese unterzogen, mittels Sybr® Gold gefärbt und auf einem Transilluminator fotografiert. Als Marker wurde ein Standardgrößenmarker (250-10.000bp) der Firma Invitrogen eingesetzt.

### Beispiel 2

### Erlangung eines STR-Vollprofils aus Hautschuppen

### Isolation der DNS

Zur Erlangung einer ausreichenden Menge an Hautschuppen wird zunächst 3h lang mit einem Luftprobenahmesystem Sartorius MD 8 Luft aus der direkten Tatortumgebung, in der Regel Raumluft, aber auch Luft aus anderen Innenräumen ist denkbar, z.B. Fahrzeuge, durch einen Nitrocellulosefilter bei 6 m³/h filtriert. Jede so erlangte Hautschuppe wird einzeln von dem Filter unter Zuhilfenahme eines Lichtmikroskops und eines Mikromanipulators unter sterilen Bedingungen in ein separates Reaktionsgefäß überführt. Nach Zugabe von 55 µl Chelex-Lösung 5% (Bio-Rad Laboratories, Hercules, CA, USA) und 3 µl Proteinase K (10IU/ml) (QIAGEN GmbH, Hilden) erfolgt die Inkubation im Thermomixer bei 37°C und 450 Upm über Nacht (ca. 14 h). Anschließend werden die Proben 8 Minuten bei 93°C aufgekocht und 15 Minuten bei 13000 Upm zentrifugiert. Der Überstand ohne Chelex-Granulat beinhaltet die zu erlangende DNS.

### STR-Multiplex-PCK

Zur Selektion auf erfolgreiche Extraktion werden 7 µL, jedes Extraktes in einer Multiplex-PCR mit dem kostengünstigen wie sensitiven Kit Q8 (hauseigenes Short Tandem Repeat-PCR-Kit der Universität Ulm, rezeptfrei verfügbar) bei 33 Zyklen nach folgendem Profil eingesetzt:

| | |
|---|---|
| Heißstart: | 11 min bei 95°C |
| Denaturierung: | 1 min bei 93°C ; 33 Zyklen |
| Annealing: | 1 min bei 59°C |
| Elongation: | 30 s bei 72°C |
| abschließende Elongation: | 40 min bei 72°C |

Beim Kit Q8 handelt es sich um einen miniSTR-Multiplex-PCR-Kit mit verkürzten Amplikons für die acht deutschen Datenbanksysteme D3S1358, FGA, TH01, VWA, SE33, D8S1179, D18S51 und D21S11 sowie den Geschlechtsmarker Amelogenin. Pro Reaktion wird folgender Ansatz für ein Gesamtvolumen von je 10,9 µL verwendet:
1,6 mM MgCl₂ (Applied Biosystems)
0,3 µg BSA (Boehringer Mannheim GmbH)
1,25 µl PCR-Buffer II (Applied Biosystems)
je 0,1 mM dNTPs
0,75 µl Primer-Mix
2U Ampli Taq Gold (Applied Biosystems)
1 - 7 µl DNS-Template
0 - 6 µl Aqua bidest.

Die Aufreinigung des PCR-Produkts erfolgt mit dem "QIAquick® PCR Purification Kit" (Qiagen, Hilden) gemäß dem Protokoll des Herstellers. Dieses System entfernt fast vollständig alle Enzyme, Salze, Oligomere, überschüssige Primer, Nukleotide und diverse weitere Fremdstoffe und konzentriert doppelsträngige DNS mit einer Länge zwischen 70 bp und 4 kb auf ein Endvolumen von 9 µl.

Für die Elektrophorese auf einem ABI Prism 3130 Genetic Analyzer (Applied Biosystems) werden je 10 µl HiDi™ Formamid (Applied Biosystems), 0,5 µl Längenstandard GeneScan™ 500 ROX Size Standard (SERAC, Bad Homburg) sowie 1 µl Amplifikat je Well eingesetzt und für 4 min bei 94°C aufgekocht. Die Elektrophorese erfolgt mit dem Polymer POP7 (Applied Biosystems) bei einer Injektionszeit von 16 s, einer Injektionsspannung von 1,2 kV und einer Laufzeit von 800 s. Zur Auswertung kann beispielsweise die Software GeneMapperID v3.2 (Applied Biosystems) verwendet werden.

Proben, in denen sich kein einziges der zu erwartenden Allele befindet, werden als leer gewertet. Proben mit mehr als zwei der zu erwartenden Allele dagegen werden als für die weiteren Untersuchungen brauchbar eingestuft. Aus Proben mit einem oder zwei der zu erwartenden Allele wird erneut 7 µl Extrakt entnommen und ein zweites Mal mit dem Q8-Kit bei 33 Zyklen angesetzt. Fand sich in diesem zweiten Durchgang nicht mindestens ein korrektes Allel, wird die Probe ebenfalls als leer gewertet, findet sich dagegen mindestens ein korrektes Allel, wird die Probe ebenfalls für die nachfolgende Weiterverarbeitung mittels RT-PCR herangezogen.

### Quantifizierung mittels RT-PCR

Für die Quantifizierung mittels RealTime-PCR wird das kommerziell verfügbare Kit Plexor® HY (Promega) auf einem Applied Biosystems 7500 RealTime-PCR System verwendet.

Der Plexor® HY beruht auf der Interaktion zwischen zwei modifizierten Nukleotiden. Einer der PCR-Primer enthält das mit einem Fluoreszenzmarker versehene Nukleotid iso-dC. Diesem lagert sich bei erfolgreicher PCR das im Reaktionsmix enthaltene, ebenfalls modifizierte Dabcyl-iso-dGTP an und führt bei Einbau in den komplementären DNA Strang zu einer Abnahme der Fluoreszenz. Durch die Verwendung verschiedener Farbstoffe und Primer-Sets ist gleichzeitig die Quantifizierung humaner DNS und humaner männlicher DNS sowie eine Abschätzung möglicher Inhibitoren durch eine Interne PCR Kontrolle (IPC) möglich. Zielsequenz des mit Flourescein als Marker versehenen autosomalen Primers ist eine 99 bp lange multicopy-Sequenz auf Chromosom 17 im humanen RNU2-Locus, der für ein an der pre-mRNA-Prozessierung beteiligten snRNA codiert. Der Y-chromosomale Primer ist CAL Fluor® Orange 560-markiert und amplifiziert eine 133 bp lange Sequenz des hodenspezifischen Proteins, Teil des YSPY-Locus. CAL Fluor® RED 610 ist der Fluoreszenzmarker für die IPC, IC5 ist als vierter Farbstoff ebenfalls in allen Wells als Passivreferenz enthalten. Die von 6,4 pg bis 100 ng reichende Standardreihe ermöglicht eine sensitive wie valide Quantifizierung im genannten Intervall. Die Durchführung folgt den Vorgaben des technischen Handbuchs bei einer Erhöhung des Probenextraktvolumens von 2 auf 3 µl entsprechend. Entsprechend wird pro Reaktion 10 µl Master Mix, 1 µl Primer/IPC Mix, 6 µl Aqua bidest. und 3 µl Template-DNS angesetzt. Jede Probe wird per Doppelbestimmung bei zwei Standardreihen und zwei Leerkontrollen je Lauf quantifiziert. Das PCR-Programm besteht aus einer initialen Denaturierung bei 95°C über 2 min mit darauf folgenden 38 Zyklen aus einer 5 sec Denaturierung bei 95°C und einer 35 sec Annealing- und Elongationsphase bei 60°C. Eine dem PCR-Programm folgende Schmelzkurve ermöglicht die Kontrolle der Spezifität der entstandenen Produkte.

## Patentansprüche

1. Ein Verfahren zur Analyse von Nukleinsäuremolekülen aus luftgetragenem biogenen Material, das Verfahren umfassend:
Filtrieren und/oder Sammeln von Luft mittels eines Luftprobennahmesystems, das einen Luftfilter und/oder ein Sammelmedium umfasst, so dass die Nukleinsäuremoleküle auf dem Luftfilter und/oder im Sammelmedium zurückbleiben;
Isolierung der Nukleinsäuremoleküle vom Luftfilter und/oder aus dem Sammelmedium; und
Analyse der Nukleinsäuremoleküle, vorzugsweise mittels PCR und anschließender Gelelektrophorese,
wobei der Filter / das Sammelmedium kontaktfrei mittels Laser oder einer mechanischen Vorrichtung in einer steril eingehausten Einheit in Einzelfragmente zerlegt wird und die Einzelfragmente manuell oder automatisch der Aufarbeitung zugeführt werden.

2. Das Verfahren gemäß Anspruch 1, wobei die Analyse mittels PCR und anschließender MALDI-TOF-Massenspektrometrie und/oder mittels *in-situ-*PCR erfolgt.

3. Das Verfahren gemäß irgendeinem der vorhergehenden Ansprüche, wobei das Luftprobennahmesystem ein Sartorius MD 8 System, ein Impaktionssystem oder ein elektrostatisches System ist.

4. Das Verfahren gemäß irgendeinem der vorhergehenden Ansprüche, wobei das Verfahren zum Nachweis kontaminierender Nukleinsäuremoleküle in Laborräumen, Produktionsräumen, Lagerräumen, Aufenthalts und Versammlungsräumen dient.

5. Das Verfahren gemäß irgendeinem der Ansprüche 1-4, wobei das Verfahren zur Gewinnung eines menschlichen DNS-Profils aus luftgetragenem menschlichem Zellmaterial dient.

6. Das Verfahren gemäß Anspruch 5, wobei das Verfahren zur forensischen Analyse von Tatorten dient.

7. Das Verfahren gemäß Anspruch 5 oder 6, wobei das Zellmaterial Hautschuppen oder Schleimhautzellen ist.

8. Verwendung eines Luftprobennahmesystems, das ein Sammelmedium und/oder einen Luftfilter umfasst, zur Analyse von Nukleinsäuremolekülen aus luftgetragenem biogenen Material, wobei der Filter / das Sammelmedium kontaktfrei mittels Laser oder einer mechanischen Vorrichtung in einer steril eingehausten Einheit in Einzelfragmente zerlegt wird und die Einzelfragmente manuell oder automatisch der Aufarbeitung zugeführt werden.

9. Verwendung eines Luftprobennahmesystems, das ein/en Sammelmedium und/oder Luftfilter umfasst, zur Gewinnung eines menschlichen DNS-Profils aus luftgetragenem menschlichem Zellmaterial, wobei der Filter / das Sammelmedium kontaktfrei mittels Laser oder einer mechanischen Vorrichtung in einer steril eingehausten Einheit in Einzelfragmente zerlegt wird und die Einzelfragmente manuell oder automatisch der Aufarbeitung zugeführt werden.

## Claims

1. A method for analyzing nucleic acid molecules from airborne biogenic material, the method comprising:
filtering and/or collecting air by means of an air sample collection system comprising an air filter and/or a collection medium in such a way, that the nucleic acid molecules remain on the air filter and/or in the collection medium;
isolating the nucleic acid molecules from the air filter and/or from the collection medium; and
analyzing the nucleic acid molecules, preferably by PCR and subsequent gel electrophoresis,
wherein the filter/collection medium is contact-free disassembled into single fragments by laser or a mechanical device in a sterily sealed unit and the single fragments are manually or automatically fed into processing.

2. The method according to claim 1, wherein the analysis is performed by PCR and subsequent MALDI-TOF mass spectrometry and/or by *in-situ* PCR.

3. The method according to any of the previous claims, wherein the air sample collection system is a Sartorius MD 8 System, an impaction system or an electrostatic system.

4. The method according to any of the previous claims, wherein the method is for detection of contaminating nucleic acid molecules in laboratory rooms, production rooms, storage rooms, recreation and assembly rooms.

5. The method according to any of the claims 1-4, wherein the method is for acquisition of a human DNA profile from airborne human cell material.

6. The method according to claim 5, wherein the method is for forensic analysis of crime scenes.

7. The method according to claim 5 or 6, wherein the cell material is skin scales or mucosal cells.

8. Use of an air sample collection system comprising a collection medium and/or an air filter for analyzing nucleic acid molecules from airborne biogenic material, wherein the filter / collection medium is disassembled into single fragments by laser or a mechanical device in a sterily sealed unit and the single fragments are manually or automatically fed into processing.

9. Use of an air sample collection system comprising a collection medium and/or an air filter for acquiring a human DNA profile from airborne human cell material, wherein the filter / collection medium is contact-free disassembled in single fragments by laser or a mechanical device in a sterily sealed unit and the single fragments are manually or automatically fed into processing.

## Revendications

1. Procédé d'analyse de molécules d'acides nucléiques à partir d'une matière biogène portée par l'air, le procédé comprenant :
la filtration et/ou l'accumulation d'air au moyen d'un système de prélèvement d'échantillons d'air, qui comprend un filtre à air et/ou un support d'accumulation de telle sorte que les molécules d'acides nucléiques restent sur le filtre à air et/ou dans le support d'accumulation ;
l'isolation des molécules d'acides nucléiques par rapport au filtre à air et/ou au support d'accumulation ; et
l'analyse des molécules d'acides nucléiques, de préférence par PCR et électrophorèse sur gel consécutive,
dans lequel le filtre/le support d'accumulation est décomposé en fragments individuels sans contact au moyen d'un laser ou d'un dispositif mécanique dans une unité stérilement encoffrée et les fragments individuels sont amenés manuellement ou automatiquement à l'étape de traitement.

2. Procédé selon la revendication 1, dans lequel l'analyse s'effectue au moyen d'une PCR et d'une spectrométrie de masse MALDI-TOF consécutive et/ou au moyen d'une PCR *in situ.*

3. Procédé selon l'une quelconque des revendications précédentes, dans lequel le système de prélèvement d'échantillons est un système Sartorius MD 8, un système d'impaction ou un système électrostatique.

4. Procédé selon l'une quelconque des revendications précédentes, dans lequel le procédé sert à détecter des molécules d'acides nucléiques contaminantes dans des salles de laboratoire, des locaux de production, des espaces de stockage, des salles de repos et de réunion.

5. Procédé selon l'une quelconque des revendications 1 à 4, dans lequel le procédé sert à obtenir un profil d'ADN humain à partir d'une matière cellulaire humaine portée par l'air.

6. Procédé selon la revendication 5, dans lequel le procédé sert à l'analyse médico-légale des lieux d'un crime.

7. Procédé selon la revendication 5 ou 6, dans lequel la matière cellulaire est des squames ou des cellules muqueuses.

8. Utilisation d'un système de prélèvement d'échantillons d'air, qui comprend un support d'accumulation et/ou un filtre à air, pour l'analyse de molécules d'acides nucléiques à partir d'une matière biogène portée par l'air, dans laquelle le filtre/le support d'accumulation est décomposé en fragments individuels sans contact au moyen d'un laser ou d'un dispositif mécanique dans une unité stérilement encoffrée et les fragments individuels sont amenés manuellement ou automatiquement à l'étape de traitement.

9. Utilisation d'un système de prélèvement d'échantillons d'air, qui comprend un support d'accumulation et/ou un filtre à air, pour l'obtention d'un profil d'ADN humain à partir d'une matière cellulaire humaine portée par l'air, dans laquelle le filtre/le support d'accumulation est décomposé en fragments individuels sans contact au moyen d'un laser ou d'un dispositif mécanique dans une unité stérilement encoffrée et les fragments individuels sont amenés manuellement ou automatiquement à l'étape de traitement.
